# EUROPÄISCHE PATENTSCHRIFT

(11) **EP 2 583 818 B1**
(45) Veröffentlichungstag und Bekanntmachung des Hinweises auf die Patenterteilung: **19.03.2014**
(21) Anmeldenummer: 12006744.2
(22) Anmeldetag: 27.09.2012
(51) Int. Cl.: B32B 5/02, D01D 5/08, D01F 1/10, D04H 1/00, D04H 3/00

(54) **Spinnvlies mit Geruchsabsorber und Verfahren zu dessen Herstellung**
Spun non-woven fabric with odour absorber and method for its production
Non-tissé doté d'un absorbeur d'odeur et son procédé de fabrication

(30) Priorität: 21.10.2011 DE 102011116554
(43) Veröffentlichungstag der Anmeldung: 24.04.2013
(73) Patentinhaber: Granulat GmbH, 53842 Troisdorf (DE)
(72) Erfinder: Waeteraere, Michael, 53797 Lohmar (DE); Fengler, Eduard, 53844 Troisdorf (DE)
(74) Vertreter: Patentanwaltskanzlei Methling

(56) Entgegenhaltungen:
- EP-A1- 1 972 247
- WO-A1-2007/053790
- WO-A1-2008/058564

## Beschreibung

Die Erfindung betrifft ein Feinfaserspinnvlies auf Basis eines Polymers, insbesondere Polyolefin, insbesondere Polypropylen, wobei zur Faserherstellung der Polymer als Rohmaterial in einem Extruder erhitzt und unter hohem Druck durch eine Spinndüse gepresst wird.

Ferner betrifft die Erfindung ein mehrlagiges Verbundmaterial mit zumindest einer Trägerschicht und zumindest einer Vliesschicht sowie einen Staubsaugerfilterbeutel, welcher aus einem mehrlagigen Verbundmaterial aufweisend mehrere Filterlagen gebildet ist und einen eingefassten Anschlussbereich aufweist, sowie Hygieneartikel, die eine oder mehrere Lagen bestehend aus Feinfaserspinnvlies aufweisen und die Verwendung eines Spinnvlieses als Bestandteil in Hygieneartikeln, Staubsaugerfilterbeuteln oder Filtern.

Ferner betrifft die Erfindung ein Verfahren zur Herstellung eines Spinnvlieses auf Basis eines Polymers, insbesondere Polyolefin, insbesondere Polypropylen, wobei zur Faserherstellung der Polymer als Rohmaterial in einem Extruder erhitzt und unter hohem Druck durch eine Spinndüse gepresst wird.

Vliesmaterialien, die nachträglich auf ihrer Oberfläche mit Mitteln behandelt werden, die ein Zinksalz der Ricinolsäure enthalten, sind aus WO 2008/058564 A1, WO 2007/053790 A1 und EP 1 972 247 A1 bekannt.

Vliesstoff oder kurz Vlies ist ein textiles Flächengebilde aus einzelnen Fasern. Im Sinne der Erfindung werden die Begriffe Feinfaserspinnvlies, Spinnvlies, Vlies oder Vliesstoff unabhängig von dem konkreten Herstellungsverfahren synonym verwendet und es sind alle nichtgewebten Stoffe umfasst, die in der englischen Sprache als sogenannte "nonwoven" bezeichnet werden. Ein Vlies besteht aus lose zusammen liegenden Fasern, welche noch nicht miteinander verbunden sind. Die Festigkeit eines Vlieses beruht nur auf der fasereigenen Haftung, kann aber durch Aufarbeitung beeinflusst werden. Um dem Vlies besondere Festigkeitseigenschaften zu verleihen, kann es weiter verfestigt werden, wofür verschiedene Methoden angewandt werden können, insbesondere das Kalandrieren.

Bei einem ersten bekannten Verfahren der Faserherstellung wird ein Polymer in einem Extruder erhitzt und auf einen hohen Druck gebracht. Das Polymer wird in genauer Dosierung mittels der Spinnpumpen durch eine Matrize, die so genannte Spinndüse gepresst. Das Polymer tritt aus der Düsenplatte als feiner Faden noch in geschmolzener Form aus. Durch einen Luftstrom wird es abgekühlt und noch aus der Schmelze gestreckt. Der Luftstrom befördert die Fäden auf ein Förderband das als Sieb ausgebildet ist. Durch eine Absaugung unter dem Siebband werden die Fäden fixiert. Dieses Faden- bzw. Fasergelege ist ein Wirrlagen-Vlies, das verfestigt werden muss. Die Verfestigung kann durch zwei beheizte Kalanderwalzen durch das sogenannte Kalandrieren oder durch einen Dampfstrom erfolgen.

Bei dem sogenannten Meltblown-Verfahren wird zur Faserherstellung ebenfalls ein Polymer in einem Extruder erhitzt und auf einen hohen Druck gebracht. Das Polymer wird in genauer Dosierung mittels der Spinnpumpen durch eine Matrize, die so genannte Spinndüse gepresst. Nach dem Austritt durch die Düsenspitze wird das Polymer durch komprimierte Prozess-Heißluft gestreckt, d.h. mittels eines Hochgeschwindigkeitsheißluftstromes gestreckt. Das entstehende Mikrofaservlies wird auf einem luftdurchlässigen Siebband abgelegt. Zur Herstellung von Laminaten kann die Meltblown-Anlage um eine Abrollstation vor und hinter der Düse erweitert sein. Ein Kalander verbindet die zugeführten Materialien. So werden ein-, zwei- und mehrlagige Stoffe produziert.

Derartige Feinfaserspinnvliese oder einfach Spinnvliese (englisch allgemein: Nonwoven) sind bekannt und werden insbesondere als Filter und hier ferner in Staubsaugerfilterbeuteln eingesetzt. Um unangenehmer Gerüche zu unterdrücken ist es bekannt, Duftkapseln in den Staubsauger einzulegen. Dies führt zu einer Vermischung des unangenehmen Geruches mit dem Aroma der Duftkapsel.

Nachteilig bei den bekannten Vliesstoffen ist es, dass diese nicht geeignet sind, unangenehme Gerüche zu binden und es ferner sehr aufwändig ist, bei dem Einsatz solcher Vliesstoffe als Staubsaugerfilterbeutel zusätzlich jeweils eine Duftkapsel in den Staubsauger einzulegen.

Die Aufgabe der Erfindung ist es, ein Feinfaserspinnvlies derart weiterzubilden, dass es geeignet ist, unangenehme Gerüche zu binden, und ferner einen Staubsaugerfilterbeutel derart weiterzubilden, dass dieser geeignet ist, unangenehme Gerüche zu binden und eine einfache Handhabung ohne den Einsatz von Duftkapseln oder dergleichen gestattet.

Eine weitere Aufgabe der Erfindung ist es, ein Herstellungsverfahren zur Herstellung eines Feinfaserspinnvlieses anzugeben, um ein Feinfaserspinnvlies zu erzeugen, welches geeignet ist, unangenehme Gerüche zu binden.

Diese Aufgabe wird erfindungsgemäß durch ein Feinfaserspinnvlies gemäß Anspruch 1, und die angegeben Verwendungen und Herstellungsverfahren gemäß den Ansprüchen 10 und 11 gelöst. Vorteilhafte Weiterbildungen der Erfindung sind in den jeweiligen abhängigen Ansprüchen angegeben.

Im Sinne der Erfindung werden die Begriffe Feinfaserspinnvlies, Spinnvlies, Vlies oder Vliesstoff unabhängig von dem konkreten Herstellungsverfahren oder der Weiterverarbeitung der Fasern synonym verwendet und es sind alle nichtgewebten Stoffe umfasst, die in der englischen Sprache als sogenannte "Nonwoven" bezeichnet werden, insbesondere sind alle durch Schmelzspinnen hergestellten Fasern und hieraus erzeugte nichtgewebte Stoffe umfasst.

Besonders Vorteilhaft bei dem erfindungsgemäßen Feinfaserspinnvlies auf Basis eines Polymers, insbesondere Polyolefin, insbesondere Polypropylen, wobei zur Faserherstellung der Polymer als Rohmaterial in einem Extruder erhitzt und unter hohem Druck durch eine Spinndüse gepresst wird, ist es, dass das Material der Fasern des Vlieses Zinksalz der Ricinolsäure aufweist, insbesondere einen Gewichtsanteil von bis zu 10 % Zinksalz der Ricinolsäure aufweist.

Dabei wird dem Rohmaterial zur Faserherstellung vor dem Extrudieren im Extruder an Anteil von 1 % bis 50 % eines Kunststoffadditivs enthaltend Zinksalz der Ricinolsäure beigemischt.

Alternativ besteht auch die Möglichkeit, dass dem Rohmaterial zur Faserherstellung während des Extrudierens im Extruder ein Anteil von 1 % bis 50 % eines Kunststoffadditivs enthaltend Zinksalz der Ricinolsäure beigemischt wird.

Als Rohmaterial zur Faserherstellung können dabei sowohl natürliche Polymere, wie beispielsweise Cellulose, oder auch synthetische Polymere, insbesondere Polypropylen zum Einsatz kommen.

Erfindungsgemäß weist das Fasermaterial des Spinnvlieses ein Geruchsabsorberadditiv auf. Dabei ist Zinksalz der Ricinolsäure bzw. Zink-Ricinoleat als Geruchsabsorber besonders gut geeignet. Der verwendete Wirkstoff, d.h. das Zinksalz der Ricinolsäure bindet die Verursacher der schlechten Gerüche, wie Stickstoff- und Schwefelverbindungen fest in einer Matrix ein und verhindert so, dass Gerüche entstehen und nach außen freigesetzt werden können.

Eingesetzt werden können die erfindungsgemäßen Feinfaserspinnvliese dabei universell, insbesondere in solchen Anwendungen, in denen sie als Filtermaterial, in Filtern und Filtereinsätzen wie beispielsweise als Bestandteil von Staubsaugerfilterbeuteln eingesetzt werden und generell in Anwendungen, bei denen es gewünscht ist, dass unangenehme Gerüche gebunden werden.

Ferner können die erfindungsgemäßen Feinfaserspinnvliese in Hygieneartikeln, wie Windeln, Binden, Tampons, Inkontinenzeinlagen und dergleichen zum Einsatz kommen, um unangenehme Gerüche zu binden.

Vorzugsweise weist das Material der Fasern des Vlieses einen Gewichtsanteil von 0,1 % bis zu 10 %, insbesondere von 0,5 % bis zu 1,5 %, insbesondere von bis zu 2 % oder bis zu 3 % oder bis zu 4 % oder bis zu 5 % oder bis zu 6 % oder bis zu 7 % oder bis zu 8 % oder bis zu 9 % Zinksalz der Ricinolsäure auf.

Es hat sich gezeigt, dass ein solcher Gewichtsanteil von Zinksalz der Ricinolsäure an dem Material der Fasern des Vlieses eine besonders gute Eignung des Vlieses zur Absorbierung unangenehmer Gerüche bewirkt. Ferner hat sich gezeigt, dass die geruchsabsorbierende Wirkung mit steigendem Anteil an Zinksalz der Ricinolsäure an dem Material der Fasern des Vliesstoffes ansteigt. Mit steigendem Anteil an Zinksalz der Ricinolsäure an dem Material der Fasern des Vliesstoffes steigt sowohl die Wirksamkeit der Bindung unangenehmer Gerüche, als auch die mögliche Einsatzdauer des Vliesstoffes, bevor die Geruchsbindungswirkung merklich absinkt.

Bevorzugt wird dem Rohmaterial zur Faserherstellung vor dem Extrudieren im Extruder an Anteil von 1 % bis 50 % eines Kunststoffadditivs enthaltend Zinksalz der Ricinolsäure beigemischt. Insbesondere kann dieses Kunststoffadditiv einen Gewichtsanteil von 10 % bis 20 %, insbesondere von 15 % Zinksalz der Ricinolsäure aufweisen.

Alternativ besteht auch die Möglichkeit, dass dem Rohmaterial zur Faserherstellung während des Extrudierens im Extruder ein Anteil von 1 % bis 50 % eines Kunststoffadditivs enthaltend Zinksalz der Ricinolsäure beigemischt wird, wobei das Kunststoffadditiv einen Gewichtsanteil von 10 % bis 20 %, insbesondere 15 % Zinksalz der Ricinolsäure aufweist.

Es besteht somit die Möglichkeit, ein Kunststoffadditiv bereitzustellen, welches einen höheren Gewichtsanteil an Zinksalz der Ricinolsäure aufweist und dieses dem Rohmaterial zur Faserherstellung beizumischen. Dabei besteht die Möglichkeit, die Beimischung vor der Zufuhr zum Extruder durchzuführen. Alternativ besteht auch die Möglichkeit, die Beimischung des Kunststoffadditivs im Extruder vorzunehmen, indem dem Extruder ein Anteil des Rohmaterials zur Faserherstellung zugeführt wird und gleichzeitig ein geringerer Massenstrom an beizumischendem Kunststoffadditiv dem Extruder zugeführt wird.

In einer bevorzugten Weiterbildung der Erfindung weist das Material der Fasern des Vlieses Aromastoffe auf, insbesondere einen Gewichtsanteil von bis zu 1,5 % Aromastoffe.

Durch die Einbringung von Aromastoffen zusätzlich zu dem geruchsbindendem Zinksalz der Ricinolsäure kann ein Vliesstoff erzeugt werden, der einerseits unangenehme Gerüche durch das Binden der die Gerüche erzeugenden Stickstoff- und Schwefelverbindungen bindet, und gleichzeitig durch die im Material der Fasern des Vliesstoffes enthaltenen Aromastoffe ein angenehmes Aroma verströmt. Dabei können beliebige Aromen verwendet werden.

Vorzugsweise erfolgt nach dem Extrudieren im Extruder und dem Spinnen der Fasern eine Verfestigung der Fasern durch Kalandrieren zwischen zwei Kalanderwalzen. Dabei sind die Kalanderwalzen beheizt und die Fasern werden angeschmolzen, so dass das Feinfaserspinnvlies verfestigt wird und die gewünschten mechanischen Eigenschaften aufweist. Bei der Verfestigung durch einen Kalander ist meist eine der beiden Walzen mit einer Gravur versehen. An den Kontaktpunkten verschmelzen die Fasern und bilden somit einen festen Vliesstoff, der die gewünschten mechanischen Eigenschaften aufweist und dazu führt, dass der Vliesstoff für eine Vielzahl von Verwendungen, wie beispielsweise als Filtermaterial geeignet ist.

Besonders vorteilhaft bei dem erfindungsgemäßen mehrlagigen Verbundmaterial, mit zumindest einer Trägerschicht und zumindest einer Vliesschicht, ist es, dass zumindest eine Lage des mehrlagigen Verbundmaterials durch ein Feinfaserspinnvlies gemäß der Erfindung gebildet ist.

Durch Einbau einer erfindungsgemäßen Feinfaserspinnvlieslage in ein mehrlagiges Verbundmaterial, beispielsweise verbunden mit einer Folie oder einer Trägerschicht oder dergleichen, können verschiedenste Anwendungen abgedeckt und realisiert werden.

Besonders vorteilhaft bei dem erfindungsgemäßen Staubsaugerfilterbeutel, welcher aus einem mehrlagigen Verbundmaterial aufweisend mehrere Filterlagen gebildet ist und einen eingefassten Anschlussbereich aufweist, ist es, dass zumindest eine Filterlage durch ein erfindungsgemäßes Spinnvlies gebildet ist.

Hierdurch ist der Staubsaugerfilterbeutel insbesondere geeignet, unangenehme Gerüche zu binden, d.h., dass der Staubsaugerbeutel nach der Benutzung weniger unangenehme Gerüche freisetzt, als dies nach dem vorbekannten Stand der Technik der Fall ist. Die Bindung der Gerüche beruht auf der beschriebenen Wirkung des Zinksalzes der Ricinolsäure, Stickstoff- und Schwefelverbindungen in einer Matrix fest einzubinden und hierdurch zu verhindern, dass Gerüche entstehen und nach außen dringen.

Vorzugsweise weist das Verbundmaterial des Staubsaugerfüterbeutels zumindest eine Grobfilterlage und eine Feinfilterlage auf.

Der Anschlussbereich des Staubsaugerfilterbeutels, welcher zum Einsetzen in den Staubsauger und zum Anschluss an den Staubsaugerschlauch dient, kann durch einen Kunststoffeinsatz oder einen Einsatz aus Fasermaterial, insbesondere Pappe, oder einen Verbund aus Kunststoff und Fasermaterial gebildet sein, wobei dieser Anschlussbereich an den das den Beutel bildende Verbundmaterial angeklebt und/oder kraftschlüssig geklemmt ist.

Besonders vorteilhaft bei dem Hygieneartikel, insbesondere Windel, Binde, Tampon, Einlage oder dergleichen, der eine oder mehrere Lagen bestehend aus Feinfaserspinnvlies aufweist, ist es, dass zumindest eine Lage durch ein Spinnvlies nach einem der Ansprüche 1 bis 6 gebildet ist.

Hierdurch ist der Hygieneartikel insbesondere geeignet, unangenehme Gerüche zu binden. Sofern das Material des in dem Hygieneartikel zum Einsatz kommenden Vlieses gleichzeitig Aromastoffe aufweist, können derartige Hygieneartikel gleichzeitig neben der Wirkung der Bindung unangenehmer Gerüche angenehme Aromen freisetzen und hierdurch eine deutlich angenehmere Anwendung für den Benutzer ermöglichen.

Besonders vorteilhaft bei dem Filter, insbesondere Filtereinsatz, insbesondere mehrlagigen Filter ist es, dass der Filter oder zumindest eine Lage des mehrlagigen Filters durch ein Spinnvlies gemäß der Erfindung gebildet ist.

Hierdurch können beispielsweise Filter für Dunstabzugshauben, Pollenfilter für Kraftfahrzeuge, Staubfiltermasken und dergleichen dergestalt ausgerüstet werden, dass unangenehme Gerüche unmittelbar durch das Filtermaterial selbst gebunden werden. Bei mehrlagigen Filtern kann eine oder mehrere Lagen als erfindungsgemäßen Vliesstoffen gebildet sein.

Besonders vorteilhaft ist eine Verwendung des erfindungsgemäßen Spinnvlieses als Bestandteil in Hygieneartikeln, insbesondere Windeln, Binden, Tampons, Einlagen oder dergleichen, oder als Bestandteil in Staubsaugerfilterbeuteln oder als Bestandteil in Filtereinsätzen.

Besonders vorteilhaft bei dem Verfahren zur Herstellung eines Spinnvlieses auf Basis eines Polymers, insbesondere Polyolefin, insbesondere Polypropylen, wobei zur Faserherstellung der Polymer als Rohmaterial in einem Extruder geschmolzen und unter hohem Druck durch eine Spinndüse gepresst wird, ist es, dass dem Rohmaterial zur Faserherstellung während des Extrudierens im Extruder ein Anteil von 1 % bis 50 % eines Kunststoffadditivs enthaltend Zinksalz der Ricinolsäure beigemischt wird, insbesondere ein Kunststoffadditiv mit einem Gewichtsanteil von 10 % bis 20 %, insbesondere 15 % Zinksalz der Ricinolsäure.

Bei einem weiteren Verfahren zur Herstellung eines Spinnvlieses auf Basis eines Polymers, insbesondere Polyolefin, insbesondere Polypropylen, wobei zur Faserherstellung der Polymer als Rohmaterial in einem Extruder geschmolzen und unter hohem Druck durch eine Spinndüse gepresst wird, ist es besonders vorteilhaft, dass dem Rohmaterial vor dem Extrudieren im Extruder ein Anteil von 1 % bis 50 % eines Kunststoffadditivs enthaltend Zinksalz der Ricinolsäure beigemischt wird, insbesondere ein Kunststoffadditiv mit einem Gewichtsanteil von 10 % bis 20 %, insbesondere 15 % Zinksalz der Ricinolsäure.

Die erfindungsgemäßen Verfahren sind dabei unabhängig von dem zugrunde liegenden Herstellungs- und Spinnverfahren zur Herstellung der Fasern anwendbar, d.h. bei allen grundsätzlich bekannten Faserherstellungsverfahren zur Produktion von nichtgewebten Stoffen, Vliesen und Vliesstoffen, sogenannter Nonwoven. Insbesondere sind die Verfahren sowohl anwendbar auf das Feinfaserspinnvliesverfahren, bei dem die Faserverlängerung nach dem Pressen der Schmelze durch die Spinndüse im freien Fall erfolgt, als auch beim sogenannten Meltblown-Verfahren, bei dem die Faserverlängerung nach dem Pressen der Schmelze durch die Spinndüse in einem Heißluftstrom erfolgt.

Die erfindungsgemäßen Verfahren sind anwendbar bei allen Verfahren zur Faserherstellung, bei denen die Faserherstellung durch Schmelzspinnen erfolgt, insbesondere unabhängig davon, wie nach dem Pressen der Schmelze durch die Spinndüse die Streckung der Fasern erfolgt. Eine Streckung der Fasern nach dem Pressen der Schmelze durch die Spinndüse kann im freien Fall und/oder in einem Heißluftstrom nach dem sogenannten Meltblown-Verfahren erfolgen.

Durch die Erfindung ist es auf besonders einfache Weise möglich, dem Fasermaterial des Feinfaserspinnvlieses Zinksalz der Ricinolsäure als Geruchsabsorber in der gewünschten Menge zuzuführen, so dass das Fasermaterial nach Abschluss des Herstellungsverfahrens den gewünschten Anteil an Zinksalz der Ricinolsäure als Geruchsabsorber aufweist. Es wird somit zur Herstellung des Feinfaserspinnvlieses ein Kunststoffadditiv enthaltend einen Geruchsabsorber in Form von Zinksalz der Ricinolsäure bereitgestellt und in der entsprechenden Menge beigemischt, so dass das Endmaterial des Feinfaserspinnvlieses den gewünschten Gewichtsanteil an Zinksalz der Ricinolsäure aufweist, insbesondere einen Gewichtsanteil von 0,15 % bis zu 10 % Zinksalz der Ricinolsäure aufweist.

Zur Herstellung wird demnach einerseits das Rohmaterial zur Herstellung der Fasern bereitgestellt, beispielsweise Polypropylen. Ferner wird ein Kunststoffadditiv bereitgestellt, welches Zinksalz der Ricinolsäure in einer höheren Konzentration aufweist, als es das Endprodukt aufweisen soll. Das Rohmaterial und das Additiv werden dann in einem festgelegten Mischungsverhältnis dem Extruder zugeführt, sodass das Endprodukt, also die Fasern des Vlieses, den gewünschten Gewichtsanteil an Zinksalz der Ricinolsäure aufweist.

Es ist dabei beliebig, ob das Additiv, welches das Zinksalz der Ricinolsäure enthält, vor dem Einfüllen in den Extruder dem Rohmaterial oder erst im Extruder dem Rohmaterial beigegeben wird, da der Vorgang des Extrudierens eine optimale und homogene Materialmischung herbeiführt und das erzeugten Fasern eine homogene Mischung des Polymers, insbesondere Polypropylen, und des Zinksalzes der Ricinolsäure aufweisen.

Dabei hat sich gezeigt, dass die geruchsabsorbierende Wirkung mit steigendem Anteil an Zinksalz der Ricinolsäure an dem Material der Fasern des Vliesstoffes ansteigt. Mit steigendem Anteil an Zinksalz der Ricinolsäure an dem Material der Fasern des Vliesstoffes steigt sowohl die Wirksamkeit der Bindung unangenehmer Gerüche, als auch die mögliche Einsatzdauer des Vliesstoffes, bevor die Geruchsbindungswirkung merklich absinkt.

Besonders vorteilhaft ist dabei, dass das Material der Fasern des Vlieses eine homogene Verteilung des geruchsbindenden Wirkstoffes aufweisen, und hierdurch ein versehentliches Abwaschen oder Abtragen des Wirkstoffes von den Fasern vermieden wird, was bei lediglich beschichteten Fasern passieren kann, verbunden mit der Folge, dass die geruchsbindende Wirkung entfällt. Bevorzugt werden dem Rohmaterial vor oder während des Extrudierens im Extruder Aromastoffe beigemischt, insbesondere ein Anteil von bis zu 1,5 % Aromastoffe.

Durch die Einbringung von Aromastoffen zusätzlich zu dem geruchsbindendem Zinksalz der Ricinolsäure kann ein Vliesstoff erzeugt werden, der einerseits unangenehme Gerüche durch das Binden der die Gerüche erzeugenden Stickstoff- und Schwefelverbindungen bindet, und gleichzeitig durch die im Material der Fasern des Vliesstoffes enthaltenen Aromastoffe ein angenehmes Aroma verströmt. Dabei können beliebige Aromen verwendet werden.

Sofern das Material des Vlieses gleichzeitig Aromastoffe aufweist, können hieraus gebildete Artikel wie beispielsweise Hygieneartikel gleichzeitig neben der Wirkung der Bindung unangenehmer Gerüche angenehme Aromen freisetzen und hierdurch eine deutlich angenehmere Anwendung für den Benutzer ermöglichen.

Vorzugsweise erfolgt nach dem Extrudieren und Spinnen der Fasern eine Verfestigung der Fasern durch Kalandrieren zwischen zwei Kalanderwalzen.

Dabei sind die Kalanderwalzen beheizt und es erfolgt ein punktuelles Aufschmelzen des Fasermaterials, wobei an den Kontaktpunkten die Fasern verschmelzen und der Vliesstoff verfestigt wird.

Hierdurch werden dem Spinnvlies die gewünschte Festigkeit und die gewünschten mechanischen Eigenschaften verliehen, sodass das Spinnvlies für eine Vielzahl von Anwendungen, beispielsweise als Filtermaterial, geeignet ist.

Das erfindungsgemäße Feinfaserspinnvlies ist somit aus Fasern gebildet, deren Material aus bis zu 10 % Gewichtsanteil Zinksalz der Ricinolsäure und bis zu 1,5 % Gewichtsanteil Aromastoffen bestehen, wobei der restliche Gewichtsanteil durch ein Polymer, insbesondere Polyolefin, bevorzugt Polypropylen, gebildet ist.

### Ausführungsbeispiele

### Beispiel 1:

Bei einem ersten Ausführungsbeispiel wurden dem Extruder Polypropylen als Rohmaterial und ein Zinksalz der Ricinolsäure enthaltendes Kunststoffadditiv in Granulatform im Verhältnis 9 zu 1 zugeführt, wobei das Kunststoffadditiv einen Anteil von 15 % an Zinksalz der Ricinolsäure aufwies. Das hierdurch hergestellte Feinfaserspinnvlies wies Fasern mit einer homogenen Zusammensetzung von 1,5 % Zinksalz der Ricinolsäure und 98,5 % Polypropylen auf und eignete sich besonders gut für eine Verwendung als Filter in Staubsaugerbeuteln, der gleichzeitig eine geruchsbindende Wirkung aufwies.

### Beispiel 2:

Bei einem zweiten Ausführungsbeispiel wurden dem Extruder Polypropylen als Rohmaterial und ein Zinksalz der Ricinolsäure enthaltendes Kunststoffadditiv in Granulatform sowie Aromastoffe zugeführt, wobei das Kunststoffadditiv einen Anteil von 15 % an Zinksalz der Ricinolsäure aufwies. Das hierdurch hergestellte Feinfaserspinnvlies wies Fasern mit einer homogenen Zusammensetzung von 3,2 % Zinksalz der Ricinolsäure und 96,8 % Polypropylen auf. Es zeigte sich eine gegenüber dem ersten Ausführungsbeispiel verbesserte geruchsbindende Wirkung des Vlieses.

### Beispiel 3:

Bei einem dritten Ausführungsbeispiel wurden dem Extruder Polypropylen als Rohmaterial und ein Zinksalz der Ricinolsäure enthaltendes Kunststoffadditiv in Granulatform im Verhältnis von 1 zu 1 zugeführt, wobei das Kunststoffadditiv einen Anteil von 15 % an Zinksalz der Ricinolsäure aufwies. Das hierdurch hergestellte Feinfaserspinnvlies wies Fasern mit einer homogenen Zusammensetzung von 8 % Zinksalz der Ricinolsäure 92 % Polypropylen auf und zeigte eine gegenüber dem zweiten Ausführungsbeispiel weiter gesteigerte geruchsbindende Wirkung.

### Beispiel 4:

Bei einem vierten Ausführungsbeispiel wurden dem Extruder Polypropylen als Rohmaterial und ein Zinksalz der Ricinolsäure enthaltendes Kunststoffadditiv in Granulatform im Verhältnis von 1 zu 1 zugeführt, wobei das Kunststoffadditiv einen Anteil von 15 % an Zinksalz der Ricinolsäure aufwies. Ferner wurden Aromastoffe in der Größenordnung von 1 % der Gesamtmenge von Rohmaterial und Additiv beigemischt. Das hierdurch hergestellte Feinfaserspinnvlies wies Fasern mit einer homogenen Zusammensetzung von 7,6 % Zinksalz der Ricinolsäure, 0,9 % Aromastoffe und 91,5 % Polypropylen auf und zeigte in etwa die selbe geruchsbindende Wirkung wie das dritte Ausführungsbeispiel.

### Beispiel 5:

Bei einem fünften Ausführungsbeispiel wurden dem Extruder Polypropylen als Rohmaterial und ein Zinksalz der Ricinolsäure enthaltendes Kunststoffadditiv in Granulatform im Verhältnis 1 zu 1 zugeführt, wobei das Kunststoffadditiv einen Anteil von 20 % an Zinksalz der Ricinolsäure aufwies. Das hierdurch hergestellte Feinfaserspinnvlies wies Fasern mit einer homogenen Zusammensetzung von 10 % Zinksalz der Ricinolsäure und 90 % Polypropylen auf und eignete sich aufgrund des relativ hohen Anteils an Zinksalz der Ricinolsäure besonders gut für eine Verwendung als Bestandteil in Hygieneartikeln wie Windeln, Binden, Tampons und dergleichen und zeigte eine sehr starke geruchsbindende Wirkung.

### Beispiel 6:

Bei einem sechsten Ausführungsbeispiel wurden dem Extruder Polypropylen als Rohmaterial und ein Zinksalz der Ricinolsäure enthaltendes Kunststoffadditiv in Granulatform im Verhältnis 1 zu 1 sowie Aromastoffe zugeführt, wobei das Kunststoffadditiv einen Anteil von 20 % an Zinksalz der Ricinolsäure aufwies. Das hierdurch hergestellte Feinfaserspinnvlies wies Fasern mit einer homogenen Zusammensetzung von 9,9 % Zinksalz der Ricinolsäure, 1,0 % Aromastoffe und 89,1 % Polypropylen auf und eignete sich besonders gut für eine Verwendung als Bestandteil in Hygieneartikeln wie Windeln, Binden, Tampons und dergleichen, da zusätzlich zu der sehr starken geruchsbindenden Wirkung angenehme Aromastoffe freigesetzt wurden und der olfaktorische Eindruck hierdurch sehr vorteilhaft war.

Bei allen Ausführungsbeispielen ließen sich die hergestellten Fasern ohne Probleme mittels Kalanderwalzen weiterverarbeiten und verfestigen sowie zuschneiden und konfektionieren.

## Patentansprüche

1. Feinfaserspinnvlies auf Basis eines Polymers, insbesondere Polyolefin, insbesondere Polypropylen, wobei zur Faserherstellung der Polymer als Rohmaterial in einem Extruder erhitzt und unter hohem Druck durch eine Spinndüse gepresst wird, **dadurch gekennzeichnet, dass** dem Rohmaterial vor dem Extrudieren oder während des Extrudierens im Extruder ein Anteil von 1 % bis 50 % eines Kunststoffadditivs enthaltend Zinksalz der Ricinolsäure beigemischt wird, so dass das Material der Fasern des Vlieses Zinksalz der Ricinolsäure aufweist, wobei das Material der Fasern des Vlieses einen Gewichtsanteil von 0.1% bis zu 10 % Zinksalz der Ricinolsäure aufweist.

2. Spinnvlies nach Anspruch 1, **dadurch gekennzeichnet, dass** das Material der Fasern des Vlieses einen Gewichtsanteil von 0,1 % bis zu 10 %, insbesondere von 0,5 % bis zu 1,5 %, insbesondere von 1 % Zinksalz der Ricinolsäure aufweist.

3. Spinnvlies nach Anspruch 1 oder 2, **dadurch gekennzeichnet, dass** dem Rohmaterial vor dem Extrudieren im Extruder ein Anteil von 10 % bis 20 % eines Kunststoffadditivs enthaltend Zinksalz der Ricinolsäure beigemischt wird, insbesondere dass das Kunststoffadditiv einen Gewichtsanteil von 10 % bis 20 %, insbesondere 15 % Zinksalz der Ricinolsäure aufweist.

4. Spinnvlies nach Anspruch 1 oder 2, **dadurch gekennzeichnet, dass** dem Rohmaterial während des Extrudierens im Extruder ein Anteil von 10 % bis 20 % eines Kunststoffadditivs enthaltend Zinksalz der Ricinolsäure beigemischt wird, insbesondere dass das Kunststoffadditiv einen Gewichtsanteil von 10 % bis 20 %, insbesondere 15 % Zinksalz der Ricinolsäure aufweist.

5. Spinnvlies nach einem der vorherigen Ansprüche, **dadurch gekennzeichnet, dass** das Material der Fasern des Vlieses Aromastoffe aufweist, insbesondere einen Gewichtsanteil von bis zu 1,5 % Aromastoffe aufweist.

6. Spinnvlies nach einem der vorherigen Ansprüche, **dadurch gekennzeichnet, dass** nach dem Extrudieren und Spinnen eine Verfestigung der Fasern durch Kalandrieren zwischen zwei Kalanderwalzen erfolgt

7. Mehrlagiges Verbundmaterial mit zumindest einer Trägerschicht und zumindest einer Vliesschicht, **dadurch gekennzeichnet, dass** zumindest eine Lage des mehrlagigen Verbundmaterials durch ein Spinnvlies nach einem der vorherigen Ansprüche gebildet ist.

8. Verwendung eines Spinnvlieses nach einem der Ansprüche 1 bis 6 als Lage oder Bestandteil in Hygieneartikeln, insbesondere Windeln, Binden, Tampons, Einlagen oder dergleichen, oder als Bestandteil in Staubsaugerfilterbeuteln oder als Filter, insbesondere Bestandteil von Filtern oder in Filtereinsätzen.

9. Verfahren zur Herstellung eines Spinnvlieses auf Basis eines Polymers, insbesondere Polyolefin, insbesondere Polypropylen, wobei zur Faserherstellung der Polymer als Rohmaterial in einem Extruder geschmolzen und unter hohem Druck durch eine Spinndüse gepresst wird, **dadurch gekennzeichnet, dass** dem Rohmaterial während des Extrudierens im Extruder ein Anteil von 1 bis 50 %, insbesondere ein Anteil von 10 % bis 20 % eines Kunststoffadditivs enthaltend Zinksalz der Ricinolsäure beigemischt wird, insbesondere ein Kunststoffadditiv mit einem Gewichtsanteil von 10 % bis 20 %, insbesondere 15 % Zinksalz der Ricinolsäure.

10. Verfahren zur Herstellung eines Spinnvlieses auf Basis eines Polymers, insbesondere Polyolefin, insbesondere Polypropylen, wobei zur Faserherstellung der Polymer als Rohmaterial in einem Extruder geschmolzen und unter hohem Druck durch eine Spinndüse gepresst wird, **dadurch gekennzeichnet, dass** dem Rohmaterial vor dem Extrudieren im Extruder ein Anteil von 1 bis 50 %, insbesondere ein Anteil von 10 % bis 20 % eines Kunststoffadditivs enthaltend Zinksalz der Ricinolsäure beigemischt wird, insbesondere ein Kunststoffadditiv mit einem Gewichtsanteil von 10 % bis 20 %, insbesondere 15 % Zinksalz der Ricinolsäure.

11. Verfahren nach Anspruch 9 oder 10, **dadurch gekennzeichnet, dass** dem Rohmaterial vor oder während des Extrudierens im Extruder Aromastoffe beigemischt werden, insbesondere ein Anteil von bis zu 1,5 % Aromastoffe.

12. Verfahren nach einem der Ansprüche 9 bis 11, **dadurch gekennzeichnet, dass** eine Streckung der Fasern nach dem Pressen der Schmelze durch die Spinndüse im freien Fall entlang einer Fallstrecke und/oder in einem Heißluftstrom erfolgt.

13. Verfahren nach einem der Ansprüche 9 bis 12, **dadurch gekennzeichnet, dass** nach dem Extrudieren und Spinnen eine Verfestigung der Fasern durch Kalandrieren zwischen zwei Kalanderwalzen erfolgt.

## Claims

1. Fine-fibre spun-bonded fabric based on a polymer, in particular polyolefin, more particularly polypropylene, the polymer, as the raw material, being heated in an extruder and pressed under high pressure through a spinneret in order to produce the fibres, **characterised in that**, prior to or during the extrusion process in the extruder, a 1 % to 50 % content of a plastics additive containing zinc ricinoleate is admixed with the raw material, such that the material for the fabric fibres comprises zinc ricinoleate, the material for the fabric fibres having a zinc ricinoleate content by weight of from 0.1 % to 10%.

2. Spun-bonded fabric according to claim 1, **characterised in that** the material for the fabric fibres has a zinc ricinoleate content by weight of from 0.1 % to 10 %, in particular from 0.5 % to 1.5 %, more particularly of 1 %.

3. Spun-bonded fabric according to either claim 1 or claim 2, **characterised in that**, prior to the extrusion process in the extruder, a 10 % to 20 % content of a plastics additive containing zinc ricinoleate is admixed with the raw material, in particular **in that** the plastics additive has a zinc ricinoleate content by weight of from 10 % to 20 %, more particularly of 15 %.

4. Spun-bonded fabric according to either claim 1 or claim 2, **characterised in that**, during the extrusion process in the extruder, a 10 % to 20 % content of a plastics additive containing zinc ricinoleate is admixed with the raw material, in particular **in that** the plastics additive has a zinc ricinoleate content by weight of from 10 % to 20 %, more particularly of 15 %.

5. Spun-bonded fabric according to any of the preceding claims, **characterised in that** the material for the fabric fibres comprises aromatic agents, and in particular has an aromatic agent content by weight of up to 1.5 %.

6. Spun-bonded fabric according to any of the preceding claims, **characterised in that**, after extrusion and spinning, the fibres are bonded by calendering between two calender rolls.

7. Multi-layer composite material comprising at least one backing layer and at least one fabric layer, **characterised in that** at least one layer of the multi-layer composite material is formed by a spun-bonded fabric according to any of the preceding claims.

8. Use of a spun-bonded fabric according to any of claims 1 to 6 as a layer or component in hygiene items, in particular nappies, sanitary towels, tampons, pads or the like, or as a component in vacuum cleaner filter bags or filters, in particular as a component of filters or in filter inserts.

9. Method for producing a spun-bonded fabric based on a polymer, in particular polyolefin, more particularly polypropylene, the polymer, as the raw material, being melted in an extruder and pressed under high pressure through a spinneret in order to produce the fibres, **characterised in that**, during the extrusion process in the extruder, a content of from 1 % to 50 %, in particular a content of from 10 % to 20 %, of a plastics additive containing zinc ricinoleate is admixed with the raw material, in particular a plastics additive having a zinc ricinoleate content by weight of from 10 % to 20 %, more particularly of 15 %.

10. Method for producing a spun-bonded fabric based on a polymer, in particular polyolefin, more particularly polypropylene, the polymer, as the raw material, being melted in an extruder and pressed under high pressure through a spinneret in order to produce the fibres, **characterised in that**, prior to the extrusion process in the extruder, a content of from 1 % to 50 %, in particular a content of from 10 % to 20 %, of a plastics additive containing zinc ricinoleate is admixed with the raw material, in particular a plastics additive having a zinc ricinoleate content by weight of from 10 % to 20 %, more particularly of 15 %.

11. Method according to either claim 9 or claim 10, **characterised in that**, prior to or during the extrusion process in the extruder, aromatic agents are admixed with the raw material, in particular an aromatic agent content of up to 1.5 %.

12. Method according to any of claims 9 to 11, **characterised in that** once the melt has been pressed through the spinneret, the fibres are drawn in freefall along a falling path and/or in a hot air stream.

13. Method according to any of claims 9 to 12, **characterised in that**, after extrusion and spinning, the fibres are bonded by calendering between two calender rolls.

## Revendications

1. Non tissé de fibres fines à base d'un polymère, en particulier une polyoléfine, tout particulièrement du polypropylène, dans lequel, pour la fabrication des fibres, on chauffe le polymère comme matière première dans une extrudeuse et on le refoule sous pression élevée à travers une filière, **caractérisé en ce que** l'on mélange à la matière première avant l'extrusion ou pendant l'extrusion dans l'extrudeuse une fraction de 1 % à 50 % d'un additif synthétique contenant un sel de zinc de l'acide ricinoléique de sorte que le matériau des fibres du non tissé présente du sel de zinc de l'acide ricinoléique, dans lequel le matériau des fibres du non tissé présente une fraction pondérale de 0,1 % à 10 % de sel de zinc de l'acide ricinoléique.

2. Non tissé selon la revendication 1, **caractérisé en ce que** le matériau des fibres du non tissé présente une fraction pondérale de 0,1 % à 10 %, en particulier de 0,5 % à 1,5 %, tout particulièrement de 1 % de sel de zinc de l'acide ricinoléique.

3. Non tissé selon la revendication 1 ou la revendication 2, **caractérisé en ce que** l'on mélange à la matière première avant l'extrusion dans l'extrudeuse une fraction de 10 % à 20 % d'un additif synthétique contenant un sel de zinc de l'acide ricinoléique, en particulier **en ce que** l'additif synthétique présente une fraction pondérale de 10 % à 20 %, tout particulièrement de 15 % de sel de zinc de l'acide ricinoléique,

4. Non tissé selon la revendication 1 ou la revendication 2, **caractérisé en ce que** l'on mélange à la matière première pendant l'extrusion dans l'extrudeuse une fraction de 10 % à 20 % d'un additif synthétique contenant un sel de zinc de l'acide ricinoléique, en particulier **en ce que** l'additif synthétique présente une fraction pondérale de 10 % à 20 %, tout particulièrement de 15 % de sel de zinc de l'acide ricinoléique.

5. Non tissé selon l'une quelconque des revendications précédentes, **caractérisé en ce que** le matériau des fibres du non tissé présente des aromatiques, en particulier une fraction pondérale allant jusqu'à 1,5 % d'aromatiques

6. Non tissé selon l'une quelconque des revendications précédentes, **caractérisé en ce que**, après extrusion et filage des fibres, on procède à une consolidation des fibres par calandrage entre deux cylindres de calandre.

7. Matériau composite stratifié avec au moins une couche de support et au moins une couche de non tissé, **caractérisé en ce qu'**au moins une couche du matériau composite stratifié est formée par un non tissé selon l'une quelconque des revendications précédentes,

8. Utilisation d'un non tissé selon l'une quelconque des revendications 1 à 6 comme couche ou composant dans des articles d'hygiène, en particulier des couches, des bandes, des tampons, des pansements ou analogues, ou comme composant dans des sacs filtrants d'aspirateurs de poussière, en particulier comme composant de filtres ou dans des masques à gaz.

9. Procédé de fabrication d'un non tissé à base d'un polymère, en particulier une polyoléfine, tout particulièrement du polypropylène, dans lequel, pour la fabrication des fibres, on fond le polymère comme matière première dans une extrudeuse et on le refoule sous pression élevée à travers une filière, **caractérisé en ce que** l'on mélange à la matière première pendant l'extrusion dans l'extrudeuse une fraction de 1 à 50 %, en particulier une fraction de 10 % à 20 % d'un additif synthétique contenant un sel de zinc de l'acide ricinoléique, en particulier un additif synthétique avec une fraction pondérale de 10 % à 20 %, tout particulièrement de 15 % de sel de zinc de l'acide ricinoléique.

10. Procédé de fabrication d'un non tissé à base d'un polymère, en particulier une polyoléfine, tout particulièrement du polypropylène, dans lequel, pour la fabrication des fibres, on fond le polymère comme matière première dans une extrudeuse et on le refoule sous pression élevée à travers une filière, **caractérisé en ce que** l'on mélange à la matière première avant l'extrusion dans l'extrudeuse une fraction de 1 à 50 %, en particulier une fraction de 10 % à 20 % d'un additif synthétique contenant un sel de zinc de l'acide ricinoléique, en particulier d'un additif synthétique avec une fraction pondérale de 10 % à 20 %, tout particulièrement de 15 % de sel de zinc de l'acide ricinoléique.

11. Procédé selon la revendication 9 ou la revendication 10, **caractérisé en ce que** l'on mélange à la matière première avant ou pendant l'extrusion dans l'extrudeuse des aromatiques, en particulier une fraction allantjusqu'à 1,5 % d'aromatiques.

12. Procédé selon l'une quelconque des revendications 9 à 11, **caractérisé en ce qu'**il se produit un allongement des fibres après refoulement de la masse fondue à travers la filière en chute libre le long d'une parcours de chute et/ou dans un courant d'air chaud.

13. Procédé selon l'une quelconque des revendications 9 à 12, **caractérisé en ce que**, après extrusion et filage, on procède à une consolidation des fibres par calandrage entre deux cylindres de calandre.
